# EUROPEAN PATENT APPLICATION

(11) **EP 2 950 127 A1**
(43) Date of publication of application: **02.12.2015**
(21) Application number: 13872928.0
(22) Date of filing: 25.09.2013
(51) Int. Cl.: G02B 7/28, A61B 1/00, A61B 1/04, G02B 7/36, G03B 13/36, H04N 5/225, H04N 5/232

(54) **IMAGING DEVICE AND METHOD FOR CONTROLLING IMAGING DEVICE**

(30) Priority: 28.01.2013 JP 2013012814
(71) Applicant: Olympus Corporation, Shibuya-ku Tokyo 151-0072 (JP)
(72) Inventor: YOSHINO, Koichiro, Tokyo 151-0072 (JP)
(74) Representative: Gunzelmann, Rainer
(86) International application number: PCT/JP2013/075868
(87) International publication number: WO 2014/115372

(57) **Abstract**

An imaging device includes an optical system, an image sensor (260), a lens position control section (340), and a focus control section (330), the focus control section (330) including a block setting section (331), a contrast value calculation section (332) that calculates block contrast value information, a contrast value storage section (333) that stores the block contrast value information about a reference image, a feature quantity calculation section (334), an effective block determination section (335) that acquires effective block information, an effective block information storage section (336) that stores the effective block information about the reference image, an AF area setting section (337) that sets an AF area from the effective block information about the reference image and the effective block information about the input image, and a contrast value determination section (338) that determines an image contrast value from the AF area.

## Description

### TECHNICAL FIELD

The present invention relates to an imaging device, a method for controlling an imaging device.

### BACKGROUND ART

A depth of field as deep as possible is required for an endoscope system in order to facilitate a diagnosis and a treatment performed by the user. In recent years, an image sensor having a large number of pixels has been used for the endoscope system, and the depth of field of the endoscope system has become shallow. Therefore, an endoscope system that implements an autofocus (AF) process has been proposed. When implementing a contrast AF process using an endoscope, if a bright spot due to reflection of illumination light is captured within the image, the contrast value may be affected to a large extent by the edge of the bright spot, and it may be difficult to appropriately detect the position at which tissue (observation target object) is brought into focus. When the AF process is performed while the user performs a treatment, an unintended object (e.g., forceps) may be brought into focus if a treatment tool (e.g., forceps) is captured within the image.

These problems are not limited to the endoscope system, but also occur when using an imaging device that implements an AF process that utilizes the contrast value. In order to solve these problems, Patent Documents 1 and 2 propose a method that sets a plurality of blocks to the AF area, detects a block that includes a bright spot or forceps, and performs the AF control process while excluding such a block from the AF area, for example.

### RELATED-ART DOCUMENT

### PATENT DOCUMENT

Patent Document 1: JP-A-2004-294788
Patent Document 2: JP-A-2011-139760

### SUMMARY OF THE INVENTION

### TECHNICAL PROBLEM

According to the method disclosed in Patent Documents 1 and 2, however, it is difficult to correctly perform the AF process when the position of the exclusion target object within the image has changed during the AF process (e.g., when the position of a bright spot within the image has changed due to a small motion (movement) of tissue (object) or the like, or the user has moved forceps).

Several aspects of the invention may provide an imaging device and a method for controlling an imaging device that implement an appropriate focus control process even when the position of the exclusion target object within the image has changed during the AF process.

### SOLUTION TO PROBLEM

According to one aspect of the invention, there is provided an imaging device comprising:
an optical system that includes a focus lens that adjusts an in-focus object plane position;
an image sensor that acquires an image of an object formed by the optical system;
a lens position control section that controls a focus lens position; and
a focus control section that performs an autofocus control process,
the focus control section including:
   a block setting section that sets a plurality of blocks to an input image;
   a contrast value calculation section that calculates block contrast value information that represents a contrast value of each of the plurality of blocks;
   a contrast value storage section that stores the block contrast value information about a reference image;
   a feature quantity calculation section that calculates a feature quantity of each of the plurality of blocks;
   an effective block determination section that determines whether or not each of the plurality of blocks is an effective block based on the feature quantity, and acquires determination results as effective block information, the effective block being a block that is effective for the autofocus control process;
   an effective block information storage section that stores the effective block information about the reference image;
   an autofocus area setting section that sets an autofocus area from the effective block information about the reference image and the effective block information about the input image; and
   a contrast value determination section that determines an image contrast value of the reference image from the autofocus area and the block contrast value information about the reference image, and determines the image contrast value of the input image from the autofocus area and the block contrast value information about the input image,
   the autofocus area setting section setting a set of the blocks that have been determined to be the effective block with respect to both the reference image and the input image, to be the autofocus area.

According to one aspect of the invention, the autofocus area is set using both the effective block information about the input image and the effective block information about the reference image, and the contrast value of each image is determined based on the autofocus area. Therefore, the contrast value can be calculated under identical conditions with respect to both the input image and the reference image while appropriately excluding an ineffective block (e.g., a block that includes a bright spot), and it is possible implement an appropriate AF control process, for example.

According to another aspect of the invention, there is provided a method for controlling an imaging device comprising:
acquiring an input image;
setting a plurality of blocks to the input image;
calculating block contrast value information that represents a contrast value of each of the plurality of blocks;
calculating a feature quantity of each of the plurality of blocks, determining whether or not each of the plurality of blocks is an effective block based on the calculated feature quantity, and acquiring effective block information about the input image, the effective block being a block that is effective for a focus control process;
reading the block contrast value information about a reference image from a contrast value storage section, and reading the effective block information about the reference image from an effective block information storage section, the reference image being an image captured at a focus lens position that differs in in-focus object plane position with respect to the input image at a timing that precedes a capture timing of the input image;
setting a set of the blocks that have been determined to be the effective block with respect to both the reference image and the input image, to be an autofocus area;
calculating an image contrast value of the reference image from the autofocus area and the block contrast value information about the reference image, and calculating the image contrast value of the input image from the autofocus area and the block contrast value information about the input image; and
performing the focus control process based on a comparison process that compares the image contrast value of the reference image with the image contrast value of the input image.

### BRIEF DESCRIPTION OF DRAWINGS

FIG. 1 illustrates a system configuration example of an imaging device according to one embodiment of the invention.
FIG. 2 illustrates a configuration example of a focus control section.
FIG. 3 illustrates another configuration example of a focus control section.
FIG. 4 is a flowchart illustrating the process according to the first embodiment.
FIG. 5 is a flowchart illustrating the process according to the modification of the first embodiment.
FIG. 6 is a flowchart illustrating a process when the third embodiment is applied to a single AF process.
FIG. 7 is a flowchart illustrating the process according to the second embodiment.
FIG. 8 is a flowchart illustrating a process when the third embodiment is applied to a full-time AF process.
FIG. 9 is a view illustrating the peak detection process according to the first embodiment.
FIG. 10 is a view illustrating the peak detection process according to the modification of the first embodiment.
FIG. 11 is a view illustrating a focus lens drive example (second embodiment).
FIGS. 12A and 12B are views illustrating a block setting process.
FIG. 13 is a view illustrating the relationship between a block and a coordinate value.
FIGS. 14A to 14C are views illustrating an example of effective block information, and an AF area setting example.

### DESCRIPTION OF EMBODIMENTS

Exemplary embodiments of the invention are described below. Note that the following exemplary embodiments do not in any way limit the scope of the invention laid out in the claims. Note also that all of the elements described in connection with the following exemplary embodiments should not necessarily be taken as essential elements of the invention.

### 1. Method

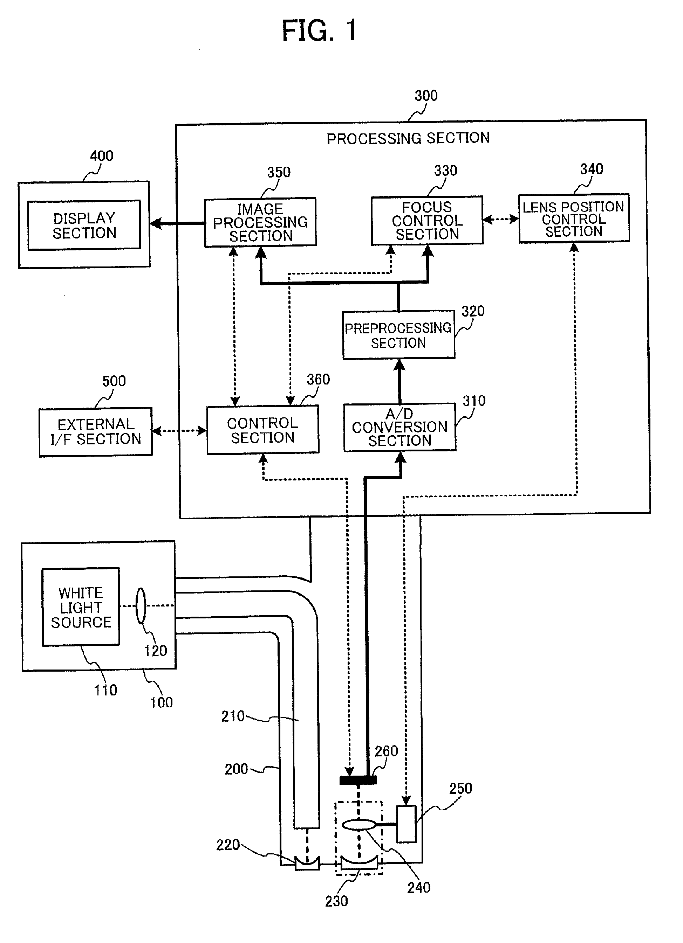

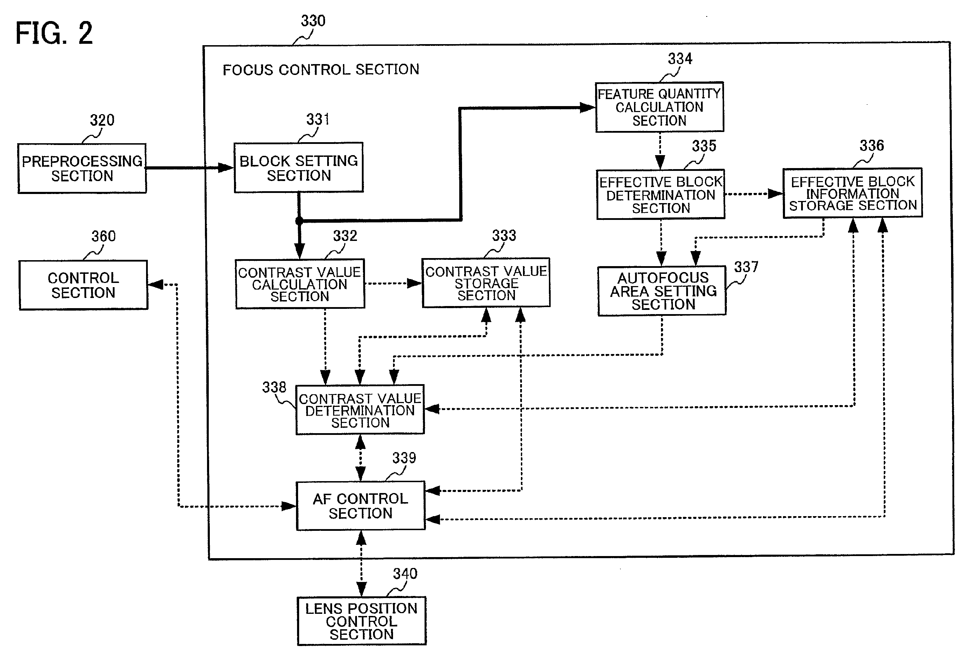

As illustrated in FIG. 1, an image processing device according to several embodiments of the invention includes an optical system that includes a focus lens 240 that adjusts the in-focus object plane position, an image sensor 260 that acquires an image of an object formed by the optical system, a lens position control section 340 that controls the position of the focus lens 240, and a focus control section 330 that performs an autofocus control process. As illustrated in FIG. 2, the focus control section 330 includes a block setting section 331 that sets a plurality of blocks to an input image, a contrast value calculation section 332 that calculates block contrast value information that represents a contrast value of each of the plurality of blocks, a contrast value storage section 333 that stores the block contrast value information about a reference image, a feature quantity calculation section 334 that calculates a feature quantity of each of the plurality of blocks, an effective block determination section 335 that determines whether or not each of the plurality of blocks is an effective block that is effective for the focus control process, based on the feature quantity, and acquires the determination results as effective block information, an effective block information storage section 336 that stores the effective block information about the reference image, an autofocus area setting section 337 that sets an autofocus area from the effective block information about the reference image and the effective block information about the input image, and a contrast value determination section 338 that determines an image contrast value of the reference image from the autofocus area and the block contrast value information about the reference image, and determines the image contrast value of the input image from the autofocus area and the block contrast value information about the input image, the autofocus area setting section 337 setting a set of blocks that have been determined to be the effective block with respect to both the reference image and the input image, to be the autofocus area.

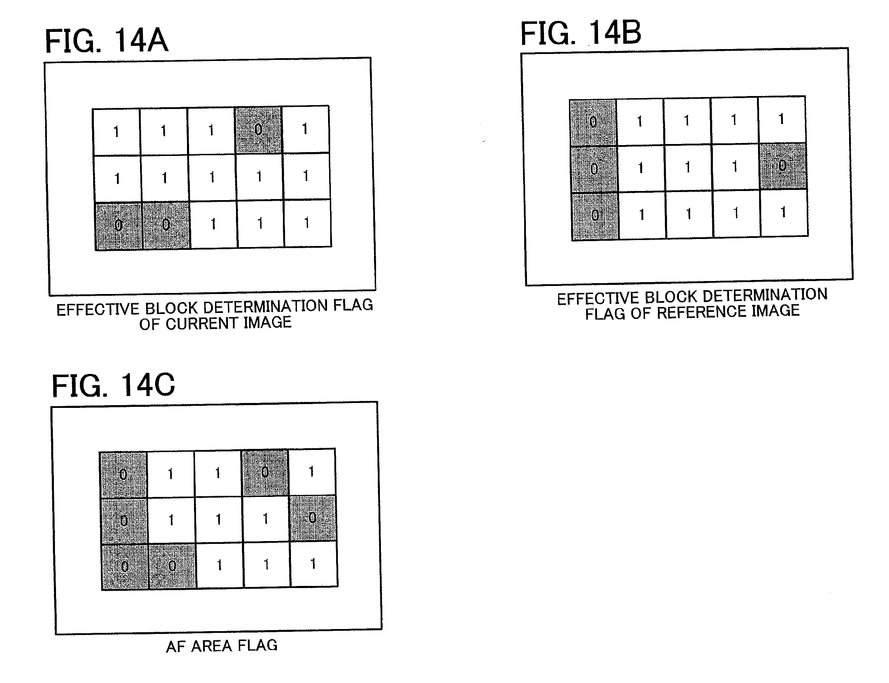

The term "in-focus object plane position" used herein refers to the position of the object when a system that includes the optical system, the image plane (i.e., the plane of the image sensor 260 in a narrow sense), and the object is in an in-focus state. The term "block contrast value information" used herein refers to information that represents a set of the block contrast values of the blocks included in the processing target image. The feature quantity is used to determine whether or not the block is the effective block. For example, the maximum brightness value, the average brightness value, the average Cr value, the average Cb value, or the like may be used as the feature quantity (described later). The term "effective block information" used herein refers to information that represents whether or not each of a plurality of blocks set to the processing target image is the effective block. The effective block information may be the information illustrated in FIGS. 14A and 14B, for example.

A method has been known that excludes a bright spot or a forceps area included in the processing target image when calculating the contrast value for implementing the AF process (see Patent Documents 1 and 2). However, the known method does not take account of a case where the position or the size of a bright spot or the like within the image changes during the AF process, for example.

Therefore, when the initial exclusion target area is continuously used during the AF process, it may be difficult to appropriately remove a bright spot or the like, and accurately calculate the contrast value when the position or the size of a bright spot or the like has changed. Even if a bright spot or the like included in each image is excluded by setting the exclusion target area within each image, since the exclusion target area (and an area used to calculate the contrast value) differs corresponding to each image during the AF operation, the contrast value is calculated from each image under different calculation conditions. Therefore, it is difficult to accurately detect the peak of the contrast value by comparing the contrast values.

In particular, when using a medical endoscope apparatus as the imaging device, since it is necessary to apply illumination light from the imaging section, and the surface of tissue (object) is wet, the position or the size of a bright spot within the image easily changes. Therefore, the above problem is not negligible.

Moreover, the known method is designed for a single AF process that determines the in-focus lens position (i.e., the focus lens position when the system is in an in-focus state) only once, and is not designed for a full-time AF process that continuously searches the in-focus lens position.

In order to solve the above problems, several embodiments of the invention propose a method that sets the autofocus area (hereinafter referred to as "AF area" (the autofocus area setting section 337 is hereinafter referred to as "AF area setting section 337")) using the effective block information (i.e., the effective block (i.e., a block other then a block that includes a bright spot or the like) determination results) about the input image, and the effective block information about the reference image, when performing the AF process on the input image. Specifically, a set of blocks that have been determined to be effective with respect to both the input image and the reference image is set to be the AF area (see FIGS. 14A to 14C). This makes it possible to calculate the contrast value of the input image and the contrast value of the reference image under identical conditions, and appropriately perform the comparison process on the input image and the reference image.

The contrast value (hereinafter may be referred to as "image contrast value" in order to distinguish this term from the term "block contrast value") of each image may be calculated by calculating the sum of the block contrast values of the blocks included in the AF area, for example.

Note that the image contrast value of an image is not necessarily identical since the AF area may change depending on the comparison target image. Specifically, the image contrast value represents the relative relationship between the reference image and the input image when performing the comparison process on the reference image and the input image, and the absolute value of the image contrast value is not necessarily important.

A first embodiment, a second embodiment, and a third embodiment of the invention are described below. The first embodiment illustrates a method that implements the single AF process, and a modification thereof, and the second embodiment illustrates a method that implements the full-time AF process. The third embodiment illustrates a method that utilizes the motion amount of the input image. Note that the method that utilizes the motion amount may be combined with the single AF process, or may be combined with the full-time AF process. The third embodiment illustrates an example in which the third embodiment is combined with the first embodiment, and an example in which the third embodiment is combined with the second embodiment.

### 2. First embodiment

The first embodiment illustrates an example in which the single AF process is performed as the focus control process. A system configuration example will be described first, and the details of the focus control section that performs the autofocus control process will then be described. The details of the process will then be described using a flowchart and the like, and a modification will be described thereafter.

### 2.1 System configuration example

An imaging device according to the first embodiment of the invention is described below with reference to FIG. 1 taking an endoscope system as a specific example. Note that the imaging device is not limited to an endoscope system. The endoscope system according to the first embodiment includes a light source section 100, an imaging section 200, a processing section 300, a display section 400, and an external I/F section 500.

The light source section 100 includes a white light source 110 that emits white light, and a condenser lens 120 that focuses the white light on a light guide fiber 210.

The imaging section 200 is formed to be elongated and flexible (i.e., can be curved) so that the imaging section 200 can be inserted into a body cavity or the like. The imaging section 200 includes the light guide fiber 210 that guides the light focused by the light source section 100, an illumination lens 220 that diffuses the light that has been guided by the light guide fiber 210, and applies the diffused light to the observation target, an objective lens system 230 that focuses the reflected light from the observation target, a focus lens 240 that is included in the objective lens system 230, and adjusts the in-focus object plane position, a lens driver section 250 that drives the focus lens 240, and an image sensor 260 that photoelectrically converts the reflected light focused by the objective lens system 230 to generate an image. The lens driver section 250 is implemented by a voice coil motor (VCM), for example. The image sensor 260 is an image sensor that includes a Bayer color filter array, for example.

The processing section 300 includes an A/D conversion section 310, a preprocessing section 320, a focus control section 330, a lens position control section 340, an image processing section 350, and a control section 360. The A/D conversion section 310 converts analog signals sequentially output from the image sensor 260 into digital image signals, and outputs the digital image signals to the preprocessing section 320. The preprocessing section 320 performs image processing (e.g., white balance process, interpolation process (demosaicing process), and YCbCr conversion process) on the image signals output from the AD conversion section 310, and sequentially outputs the resulting image signals to the focus control section 330 and the image processing section 350.

The lens position control section 340 is connected to the lens driver section 250 and the focus control section 330, and controls the focus lens 240 in accordance with information output from the focus control section 330. The details of the focus control section 330 are described later.

The image processing section 350 performs image processing (e.g., color conversion process, grayscale transformation process, edge enhancement process, and noise reduction process) on the image signals output from the preprocessing section 320, and sequentially outputs the image signals to the display section 400. The display section 400 is a liquid crystal monitor, for example. The display section 400 displays the image signals output from the image processing section 350.

The control section 360 is connected to the external I/F section 500, the focus control section 330, the image processing section 350, the image sensor 260, and the like, and controls the external I/F section 500, the focus control section 330, the image processing section 350, the image sensor 260, and the like. The external I/F section 500 is an interface that allows the user to perform an input operation or the like on the endoscope system. The external I/F section 500 includes an AF button (AF start/stop button), an adjustment button for adjusting the image processing parameter, and the like.

### 2.2 Details of focus control section

The details of the focus control section 330 are described below with reference to FIG. 2. Note that the image that is sequentially output from the preprocessing section 320 to the focus control section 330 is hereinafter referred to as "current image (input image)".

The focus control section 330 includes a block setting section 331, a contrast value calculation section 332, a contrast value storage section 333, a feature quantity calculation section 334, an effective block determination section 335, an effective block information storage section 336, an AF area setting section 337, a contrast value determination section 338, and anAF control section 339.

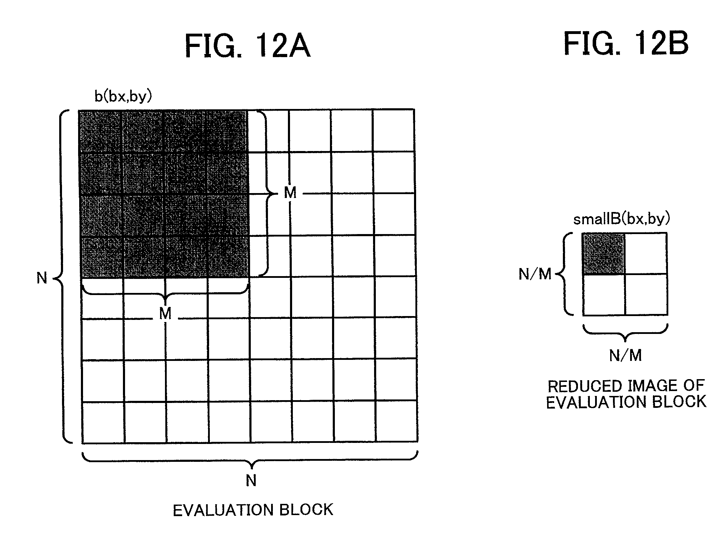

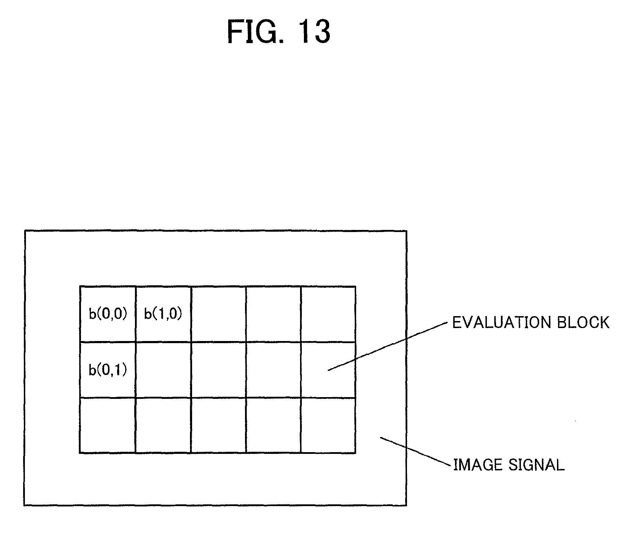

The block setting section 331 sets a plurality of evaluation blocks (see FIG. 12A) to the current image output from the preprocessing section 320. Each evaluation block is represented by b(bx, by) (see FIG. 13). Note that the coordinates(bx, by) are defined relative to the coordinates (0, 0) (reference position) of the upper left evaluation block.

The contrast value calculation section 332 calculates an evaluation block contrast value blockContrastValNow(bx, by) corresponding to each evaluation block that has been set by the block setting section 331, and outputs the calculated contrast value blockContrastValNow(bx, by) to the contrast value determination section 338 and the contrast value storage section 333. For example, a high-pass filtering process may be performed on the Y signal of each pixel included in each evaluation block, and the sum of the output values may be used as the contrast value of each evaluation block.

The contrast value storage section 333 stores the contrast value blockContrastValNow(bx, by) of each evaluation block that has been calculated by the contrast value calculation section 332 according to a control signal output from the AF control section 339, and outputs the contrast value blockContrastValNow(bx, by) to the contrast value determination section 338 as the contrast value blockContrastValRef(bx, by) of each evaluation block of the reference image. The details of the contrast value storage section 333 and the reference image are described later.

The feature quantity calculation section 334 calculates a feature quantity corresponding to each evaluation block that has been set by the block setting section 331, and outputs the calculated feature quantity to the effective block determination section 335. For example, the maximum value blockMaxY(bx, by) or the average value blockAveY(bx, by) of the Y signals of the pixels included in each evaluation block, the average value blockAveCb(bx, by) of the Cb signals of the pixels included in each evaluation block, the average value blockAveCr(bx,by) of the Cr signals of the pixels included in each evaluation block, and the like are calculated as the feature quantity, and output to the effective block determination section 335.

The effective block determination section 335 determines whether or not each evaluation block is an effective block using the feature quantity of each evaluation block output from the feature quantity calculation section 334. For example, the effective block determination section 335 determines whether or not the maximum value blockMaxY(bx, by) of each evaluation block is equal to or larger than a given threshold value. The effective block determination section 335 determines that the evaluation block is not the effective block when the maximum value blockMaxY(bx, by) is equal to or larger than the threshold value on the assumption that a bright spot is included in the evaluation block, and sets an effective block determination flag effectiveBlockFlagNow(bx, by) of the corresponding evaluation block to "0". The effective block determination section 335 determines that the evaluation block is the effective block when the maximum value blockMaxY(bx, by) is smaller than the threshold value on the assumption that a bright spot is not included in the evaluation block, and sets the effective block determination flag effectiveBlockFlagNow(bx, by) of the corresponding evaluation block to "1".

For example, the effective block determination section 335 determines whether or not the average value blockAveY(bx, by) of each evaluation block is equal to or smaller than a given threshold value. The effective block determination section 335 determines that the evaluation block is not the effective block when the average value blockAveY(bx, by) is equal to or smaller than the threshold value on the assumption that the evaluation block is situated in a very dark area of the image, and sets the effective block determination flag effectiveBlockFlagNow(bx, by) of the corresponding evaluation block to "0". The effective block determination section 335 determines that the evaluation block is the effective block when the average value blockAveY(bx, by) is larger than the threshold value on the assumption that the evaluation block is situated in a bright area of the image, and sets the effective block determination flag effectiveBlockFlagNow(bx, by) of the corresponding evaluation block to "1".

For example, the effective block determination section 335 determines whether or not both the average value blockAveCb(bx, by) and the average value blockAveCr(bx,by) of each evaluation block are equal to or smaller than a given threshold value. The effective block determination section 335 determines that the evaluation block is not the effective block when both the average value blockAveCb(bx, by) and the average value blockAveCr(bx,by) are equal to or smaller than the threshold value on the assumption that the evaluation block is situated in a forceps area of the image, and sets the effective block determination flag effectiveBlockFlagNow(bx, by) of the corresponding evaluation block to "0". Specifically, since forceps are normally black or silver, both the Cb signal and the Cr signal have a value close to 0 in a forceps area of the image. The effective block determination section 335 determines that the evaluation block is the effective block when both of the average value blockAveCb(bx, by) and the average value blockAveCr(bx,by) are larger than the threshold value, or either of the average value blockAveCb(bx, by) and the average value blockAveCr(bx,by) is larger than the threshold value, on the assumption that the evaluation block is not situated in a forceps area of the image, and sets the effective block determination flag effectiveBlockFlagNow(bx, by) of the corresponding evaluation block to "1".

The effective block determination section 335 performs one of these determination processes, or performs a plurality of determination processes among these determination processes in an arbitrary combination, and outputs the effective block determination flag effectiveBlockFlagNow(bx, by) of each evaluation block to the AF area setting section 337 and the effective block information storage section 336. When the effective block determination section 335 performs a plurality of determination processes, the effective block determination section 335 may set the effective block determination flag effectiveBlockFlagNow(bx, by) to "1" when the evaluation block has been determined to be the effective block by each determination process. The effective block determination section 335 may set the effective block determination flag effectiveBlockFlagNow(bx, by) to "0" when the evaluation block has been determined to be the ineffective block by at least one determination process.

The feature quantity calculation section 334 may optionally calculate an arbitrary feature quantity other than the above feature quantities, and the effective block determination section 335 may perform an arbitrary determination process corresponding to the calculated feature quantity to determine whether or not each evaluation block is the effective block.

The effective block information storage section 336 stores the effective block determination flag effectiveBlockFlagNow(bx, by) of each evaluation block that has been output from the effective block determination section 335 according to a control signal output from the AF control section 339, and outputs the effective block determination flag effectiveBlockFlagNow(bx, by) to the AF area setting section 337 as an effective determination flag effectiveBlockFlagRef(bx, by) of the reference image. The details of the effective block information storage section 336 and the reference image are described later.

The AF area setting section 337 sets the AF area from the effective block determination flag effectiveBlockFlagNow(bx,by) of the current image output from the effective block determination section 335, and the effective block determination flag effectiveBlockFlagRef(bx,by) of the reference image output from the effective block information storage section 336, and outputs the AF area to the contrast value determination section 338. For example, the AF area setting section 337 calculates the logical AND of the effective block determination flag effectiveBlockFlagNow(bx,by) (see FIG. 14A) and the effective block determination flag effectiveBlockFlagRef(bx,by) (see FIG. 14B) on an evaluation block basis, and outputs the calculation results to the contrast value determination section 338 as an AF area flag afAreaFlag(bx, by) (see FIG. 14C). The AF area setting section 337 can thus set a set of the evaluation blocks that have been determined to be the effective block with respect to both the current image and the reference image to be the AF area.

The contrast value determination section 338 determines the contrast value contrastValNow of the current image from the AF area flag afAreaFlag(bx,by) output from the AF area setting section 337, and the contrast value blockContrastValNow(bx,by) of each evaluation block of the current image output from the contrast value calculation section 332, and outputs the contrast value contrastValNow to the AF control section 339. The contrast value determination section 338 determines the contrast value contrastValRef of the reference image from the AF area flag afAreaFlag(bx,by) output from the AF area setting section 337, and the contrast value blockContrastValRef(bx,by) of each evaluation block of the reference image output from the contrast value storage section 333, and outputs the contrast value contrastValRef to the AF control section 339. Specifically, the contrast value determination section 338 calculates the sum of the contrast values blockContrastValNow(bx, by) corresponding to the evaluation blocks for which the AF area flag afAreaFlag(bx, by) is set to "1", and sets the calculated value to be the contrast value contrastValNow. The contrast value determination section 338 calculates the sum of the contrast values blockContrastValRef(bx,by) corresponding to the evaluation blocks for which the AF area flag afAreaFlag(bx, by) is set to "1", and sets the calculated value to be the contrast value contrastValRef. The contrast value determination section 338 can thus determine the contrast value (image contrast value) corresponding to each image using only the evaluation blocks that have been determined to be the effective block with respect to both the current image and the reference image.

The AF control section 339 performs the AF control process using the contrast value contrastValNow of the current image and the contrast value contrastValRef of the reference image output from the contrast value determination section 338 according to an AF start/stop signal output from the control section 360. In this case, the AF control section 339 controls the position of the focus lens 240 by outputting the lens position (required lens position) required for the focus lens 240 to the lens position control section 340. The AF control section 339 acquires the focus lens position from the lens position control section 340. The AF control section 339 acquires a control signal for controlling the image sensor 260 (e.g., image acquisition stop timing signal) from the control section 360.

### 2.3 Details of process

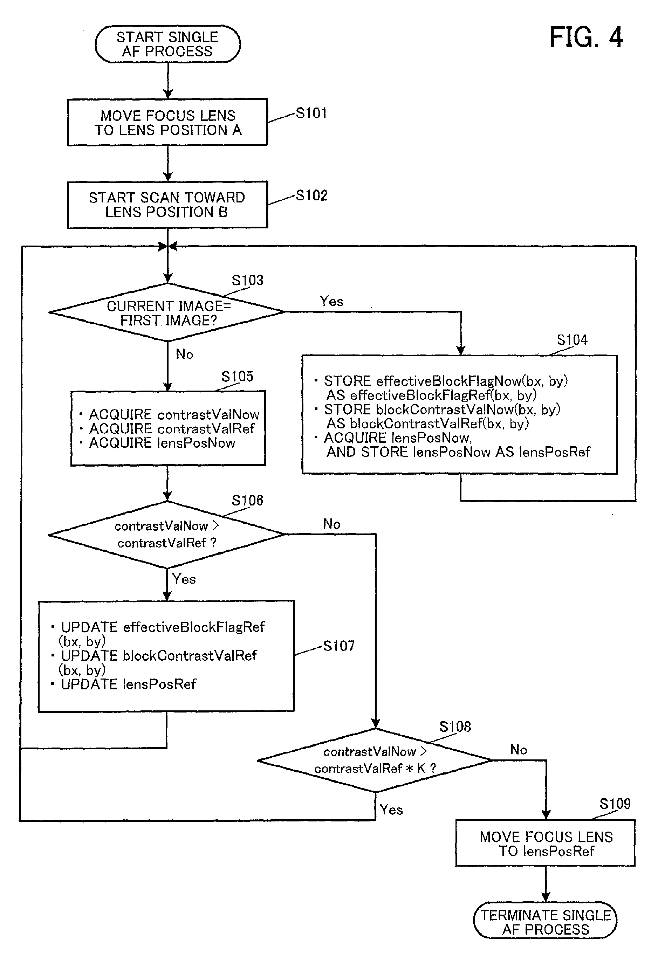

The operation of the AF control section 339 when the AF control section 339 performs the single AF process is described below with reference to FIG. 4 (flowchart). When the AF start signal has been output from the control section 360, the AF control section 339 outputs the position (lens position A) of an arbitrary end of the movable range of the focus lens 240 to the lens position control section 340 as the required lens position (S101). When the focus lens 240 has reached the lens position A, the AF control section 339 changes the required lens position to the position (lens position B) of the other end of the movable range of the focus lens 240, and causes the focus lens 240 to start a scan motion to detect the in-focus lens position (peak detection process) (S102).

The AF control section 339 determines whether or not the current image is the first image after the scan motion has started (S103). When the current image is the first image after the scan motion has started, the AF control section 339 outputs a control signal so that the effective block determination flag effectiveBlockFlagNow(bx, by) that is output from the effective block determination section 335 to the effective block information storage section 336 is stored as the effective determination flag effectiveBlockFlagRef(bx, by). The AF control section 339 outputs a control signal so that the contrast value blockContrastValNow(bx, by) that is output from the contrast value calculation section 332 to the contrast value storage section 333 is stored as the contrast value blockContrastValRef(bx,by). The AF control section 339 acquires a focus lens position lensPosNow (when the current image was acquired) from the lens position control section 340, and stores the focus lens position lensPosNow as a focus lens position lensPosRef (when the reference image was acquired) (S104). The AF control section 339 then continues the peak detection process.

When the current image is the second or subsequent image after the scan motion has started (No in S103), the AF control section 339 acquires the contrast value contrastValNow of the current image (S105). In this case, the AF control section 339 reads the stored effective determination flag effectiveBlockFlagRef(bx, by), and performs the AF area setting process and the like.

The AF control section 339 acquires the contrast value contrastValNow and the contrast value contrastValRef output from the contrast value determination section 338, and compares the contrast value contrastValNow with the contrast value contrastValRef (S106). When the contrast value contrastValNow is larger than the contrast value contrastValRef (Yes in S106), the AF control section 339 updates the effective determination flag effectiveBlockFlagRef(bx, by) so that the effective block determination flag effectiveBlockFlagNow(bx, by) that is output from the effective block determination section 335 to the effective block information storage section 336 is stored as a new effective determination flag effectiveBlockFlagRef(bx, by). The AF control section 339 outputs a control signal so that the contrast value blockContrastValNow(bx, by) that is output from the contrast value calculation section 332 to the contrast value storage section 333 is stored as a new contrast value blockContrastValRef(bx,by) to update the contrast value blockContrastValRef(bx,by). The AF control section 339 acquires the focus lens position lensPosNow from the lens position control section 340, and stores the focus lens position lensPosNow as a new focus lens position lensPosRef to update the focus lens position lensPosRef (S107). The AF control section 339 then continues the peak detection process.

When the contrast value contrastValNow is smaller than the contrast value contrastValRef (No in S106), the AF control section 339 compares the contrast value contrastValNow with a contrast value contrastValRef*K (S108). Note that K is an arbitrary coefficient within the range from 0 to 1. When the contrast value contrastValNow is larger than the contrast value contrastValRef*K, the AF control section 339 continues the peak detection process without updating the effective determination flag effectiveBlockFlagRef(bx, by), the contrast value blockcontrastValNow(bx, by), and the focus lens position lensPosRef. When the contrast value contrastValNow is equal to or smaller than the contrast value contrastValRef*K (No in S108), the AF control section 339 determines that the focus lens position lensPosRef is the peak position, and terminates the peak detection process (S109).

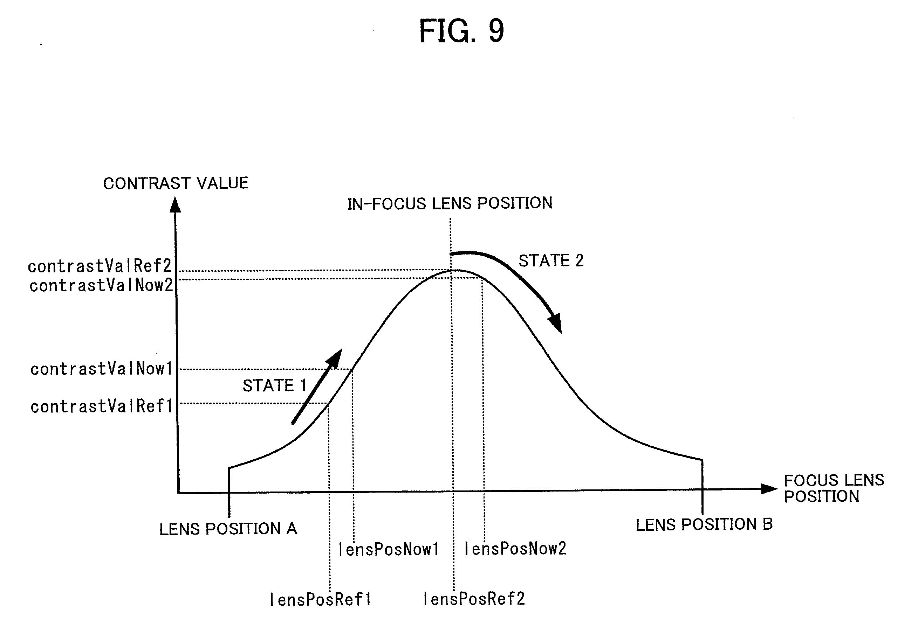

The AF control section 339 can detect the in-focus lens position as the focus lens position lensPosRef by performing the above process at a timing at which the current image is updated. The details thereof are described below with reference to FIG. 9. FIG. 9 is a schematic view illustrating a general relationship between the focus lens position and the contrast value of the image. When the focus lens 240 makes the scan motion from the lens position A toward the lens position B, the contrast value increases as the focus lens 240 moves closer to the in-focus lens position, and decreases as the focus lens 240 moves away from the in-focus lens position (see FIG. 9).

In the first embodiment, the contrast value contrastValNow and the contrast value contrastValRef corresponding to the current image and the reference image are calculated using only the evaluation blocks that have been determined to be the effective block with respect to both the current image and the reference image. Therefore, since the positions and the number of effective blocks change corresponding to the presence or absence and the position of a bright spot or forceps in the reference image and the current image, for example, the absolute value of the contrast value contrastValNow and the absolute value of the contrast value contrastValRef change. However, the relationship between the contrast value contrastValNow and the contrast value contrastValRef is determined in the same manner as in FIG. 9 corresponding to the focus lens position when the reference image was acquired, and the focus lens position when the current image was acquired.

For example, a contrast value contrastValNow1 that is acquired in a state 1 in which the focus lens position moves closer to the in-focus lens position (see FIG. 9) is larger than a contrast value contrastValRefl. In this case, the focus lens position lensPosRef is sequentially updated with the focus lens position lensPosNow (when the current image was acquired).

A contrast value contrastValNow2 that is acquired in a state 2 in which the focus lens position moves away from the in-focus lens position (see FIG. 9) is smaller than a contrast value contrastValRef2. In this case, the in-focus lens position is stored as the focus lens position lensPosRef. Since the AF control section 339 terminates the peak detection process when the difference between the in-focus lens position and the focus lens position when the current image was acquired has increased, and a contrast value contrastValNow2 has become equal to or smaller than a contrast value contrastValRef2*K, the in-focus lens position has been stored as the focus lens position lensPosRef.

The AF control section 339 can prevent erroneous peak detection, and accurately detect the in-focus lens position, even when a decrease in contrast value has occurred at a focus lens position other than the in-focus lens position due to a small motion (movement) of tissue (object) or the imaging section 200, the effects of noise, or the like, by appropriately setting the coefficient K.

After completion of the peak detection process, the AF control section 339 outputs the focus lens position lensPosRef to the lens position control section 340 as the required lens position, and terminates the single AF process when the focus lens 240 has reached the focus lens position lensPosRef (i.e., when the focus operation has been completed).

The endoscope system according to the first embodiment can thus correctly perform the single AF process even when the position of the exclusion target object within the image has changed during the AF process (e.g., when the position of a bright spot within the image has changed due to a small motion (movement) of tissue (object) or the like, or the user has moved forceps).

### 2.4 Modification

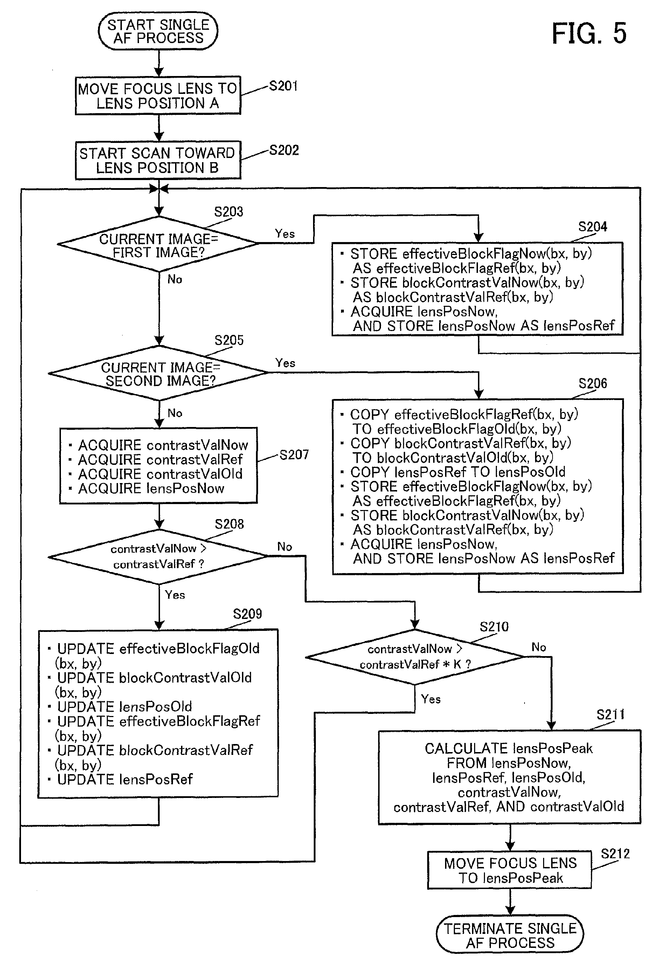

A modification of the first embodiment is described below with reference to FIG. 5 (flowchart). Note that the steps S201 and S202 are respectively the same as the steps S101 and S102 in FIG. 4. The AF control section 339 according the modification determines whether or not the current image is the first image after the scan motion has started (S203). When the current image is the first image after the scan motion has started, the AF control section 339 outputs a control signal so that the effective block determination flag effectiveBlockFlagNow(bx, by) that is output from the effective block determination section 335 to the effective block information storage section 336 is stored as the effective determination flag effectiveBlockFlagRef(bx, by). The AF control section 339 outputs a control signal so that the contrast value blockContrastValNow(bx, by) that is output from the contrast value calculation section 332 to the contrast value storage section 333 is stored as the contrast value blockContrastValRef(bx,by). The AF control section 339 acquires the focus lens position lensPosNow from the lens position control section 340, and stores the focus lens position lensPosNow as the focus lens position lensPosRef (S204). The AF control section 339 then continues the peak detection process.

The AF control section 339 determines whether or not the current image is the second or subsequent image after the scan motion has started (S205). When the current image is the second or subsequent image after the scan motion has started, the AF control section 339 outputs a control signal so that the effective determination flag effectiveBlockFlagRef(bx, by) that is stored in the effective block information storage section 336 is copied to an effective block determination flag effectiveBlockFlagOld(bx,by) of the image that precedes the reference image. The AF control section 339 outputs a control signal so that the contrast value blockContrastValRef(bx,by) that is stored in the contrast value storage section 333 is copied to a contrast value blockContrastValOld(bx,by) of each evaluation block of the image that precedes the reference image. The AF control section 339 copies the focus lens position lensPosRef to a focus lens position lensPosOld of the image that precedes the reference image. The AF control section 339 outputs a control signal so that the effective block determination flag effectiveBlockFlagNow(bx, by) that is output from the effective block determination section 335 to the effective block information storage section 336 is stored as the effective determination flag effectiveBlockFlagRef(bx, by). The AF control section 339 outputs a control signal so that the contrast value blockContrastValNow(bx, by) that is output from the contrast value calculation section 332 to the contrast value storage section 333 is stored as the contrast value blockContrastValRef(bx,by). The AF control section 339 acquires the focus lens position lensPosNow from the lens position control section 340, and stores the focus lens position lensPosNow as the focus lens position lensPosRef (S206). The AF control section 339 then continues the peak detection process.

When the current image is the third or subsequent image after the scan motion has started (No in S205), the AF control section 339 acquires the contrast value contrastValNow and the contrast value contrastValRef output from the contrast value determination section 338, and compares the contrast value contrastValNow with the contrast value contrastValRef (S207 and S208).

When the contrast value contrastValNow is larger than the contrast value contrastValRef (Yes in S208), the AF control section 339 outputs a control signal so that the effective determination flag effectiveBlockFlagRef(bx, by) that is stored in the effective block information storage section 336 is copied to the effective block determination flag effectiveBlockFlagOld(bx,by) to update the effective block determination flag effectiveBlockFlagOld(bx,by). The AF control section 339 outputs a control signal so that the contrast value blockContrastValRef(bx,by) that is stored in the contrast value storage section 333 is copied to the contrast value blockContrastValOld(bx,by) to update the contrast value blockContrastValOld(bx,by). The AF control section 339 copies the focus lens position lensPosRef to the focus lens position lensPosOld to update the focus lens position lensPosOld. The AF control section 339 outputs a control signal so that the effective block determination flag effectiveBlockFlagNow(bx, by) that is output from the effective block determination section 335 to the effective block information storage section 336 is stored as the effective determination flag effectiveBlockFlagRef(bx, by). The AF control section 339 outputs a control signal so that the contrast value blockContrastValNow(bx, by) that is output from the contrast value calculation section 332 to the contrast value storage section 333 is stored as the contrast value blockContrastValRef(bx,by). The AF control section 339 acquires the focus lens position lensPosNow from the lens position control section 340, and stores the focus lens position lensPosNow as the focus lens position lensPosRef (S209). The AF control section 339 then continues the peak detection process.

When the contrast value contrastValNow is smaller than the contrast value contrastValRef (No in S208), the AF control section 339 compares the contrast value contrastValNow with the contrast value contrastValRef*K (S210). Note that K is an arbitrary coefficient within the range from 0 to 1. When the contrast value contrastValNow is larger than the contrast value contrastValRef*K (Yes in S210), the AF control section 339 continues the peak detection process without updating the effective determination flag effectiveBlockFlagRef(bx, by), the contrast value blockcontrastValNow(bx, by), and the focus lens position lensPosRef. When the contrast value contrastValNow is equal to or smaller than the contrast value contrastValRef*K (No in S210), the AF control section 339 determines that the in-focus lens position has been detected, and terminates the peak detection process.

Note that the AF area setting section 337 according to the modification sets the AF area from the effective block determination flag effectiveBlockFlagNow(bx,by) output from the effective block determination section 335, the effective block determination flag effectiveBlockFlagRef(bx,by) output from the effective block information storage section 336, and the effective block determination flag effectiveBlockFlagOld(bx,by) output from the effective block information storage section 336, and outputs the AF area to the contrast value determination section 338. Specifically, the AF area setting section 337 calculates the logical AND of the effective block determination flag effectiveBlockFlagNow(bx,by) and the effective block determination flag effectiveBlockFlagRef(bx,by) on an evaluation block basis, calculates the logical AND of the calculation results and the effective block determination flag effectiveBlockFlagOld(bx,by) on an evaluation block basis, and outputs the calculation results to the contrast value determination section 338 as the AF area flag afAreaFlag(bx, by). The AF area setting section 337 can thus set a set of the evaluation blocks that have been determined to be the effective block with respect to the current image, the reference image (hereinafter appropriately referred to as "first reference image"), and the image that precedes the reference image (hereinafter appropriately referred to as "second reference image") to be the AF area.

The contrast value determination section 338 according to the modification determines the contrast value contrastValNow from the AF area flag afAreaFlag(bx,by) output from the AF area setting section 337, and the contrast value blockContrastValNow(bx,by) output from the contrast value calculation section 332, and outputs the contrast value contrastValNow to the AF control section 339. The contrast value determination section 338 determines the contrast value contrastValRef and the contrast value contrastValOld from the AF area flag afAreaFlag(bx,by) output from the AF area setting section 337, and the contrast value blockContrastValRef(bx,by) and the contrast value blockContrastValOld(bx,by) output from the contrast value storage section 333, and outputs the contrast value contrastValRef and the contrast value contrastValOld to the AF control section 339.

Specifically, the contrast value determination section 338 calculates the sum of the contrast values blockContrastValNow(bx, by) corresponding to the evaluation blocks for which the AF area flag afAreaFlag(bx, by) is set to "1", and sets the calculated value to be the contrast value contrastValNow. The contrast value determination section 338 calculates the sum of the contrast values contrastValRef corresponding to the evaluation blocks for which the AF area flag afAreaFlag(bx, by) is set to "1", and the sum of the contrast values contrastValOld corresponding to the evaluation blocks for which the AF area flag afAreaFlag(bx, by) is set to "1", and sets the calculated values to be the contrast value contrastValRef and the contrast value contrastValOld. The contrast value determination section 338 can thus determine the contrast value corresponding to each image using only the evaluation blocks that have been determined to be the effective block with respect to all of the current image, the reference image, and the image that precedes the reference image.

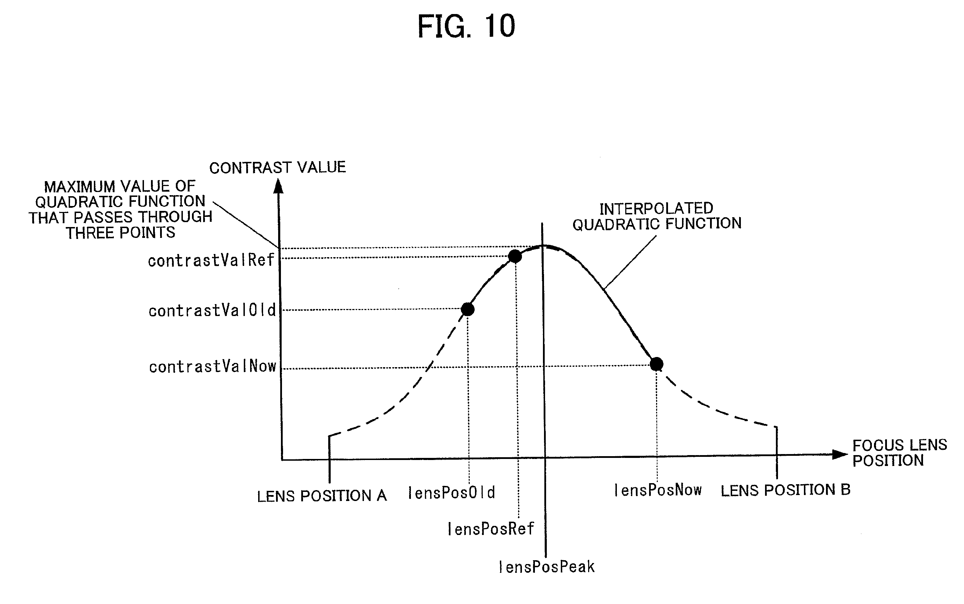

After completion of the peak detection process, the AF control section 339 calculates a quadratic function that passes through the lens position lensPosNow, the lens position lensPosRef, and the lens position lensPosOld (see FIG. 10) from the lens position lensPosNow, the lens position lensPosRef, the lens position lensPosOld, the contrast value contrastValNow, the contrast value contrastValRef, and the contrast value contrastValOld using known Lagrange interpolation or the like. The AF control section 339 determines a focus lens position lensPosPeak of the calculated quadratic function at which the contrast value becomes a maximum to be the required lens position, and outputs the focus lens position lensPosPeak to the lens position control section 340 (S211). When the focus lens 240 has reached the focus lens position lensPosPeak (i.e., when the focus operation has been completed) (S212), the AF control section 339 terminates the single AF process.

The endoscope system according to the modification can thus accurately detect the in-focus lens position, and implement a high-speed single AF process, even when the scan pitch of the focus lens 240 is increased.

As another modification, the imaging device may include a distance measurement section that calculates the distance to the object using the in-focus lens position after completion of the AF process. Since the in-focus object plane position corresponding to the focus lens position is uniquely determined from the optical characteristics, the in-focus object plane position can be calculated when it has been determined that the focus lens position is a given position. Since the focus lens position after the AF process has normally ended is the in-focus lens position at which the imaging target object is expected to be in focus, it is considered that the object is situated at a position corresponding to the in-focus object plane position. Therefore, the distance measurement section may store table data that represents the relationship between the in-focus lens position and the in-focus object plane position in a memory, and calculate the in-focus object plane position from the in-focus lens position after completion of the AF process using the table data to determine the distance to the object, for example.

Specifically, distance information that represents the distance to the object can be calculated using the AF results (particularly the focus lens position). The acquired distance information may be used for an arbitrary process. For example, the structure of the object or the like may be estimated from the distance information, and an enhancement process that improves visibility may be performed on a specific irregular (uneven) structure, or an alert may be displayed (output) when the distance indicated by the distance information is smaller than a given threshold value since the imaging section may come in contact with tissue (object).

According to the first embodiment, the imaging device includes the optical system that includes the focus lens 240 that adjusts the in-focus object plane position, the image sensor 260 that photoelectrically converts the image of the object formed by the optical system to generate an image, the lens position control section 340 that controls the position (focus lens position) of the focus lens 240, and the focus control section 330 that performs the autofocus control process (see FIG. 1). The focus control section 330 includes the block setting section 331 that sets a plurality of blocks to the input image, the contrast value calculation section 332 that calculates block contrast value information that represents the contrast value of each of the plurality of blocks, the contrast value storage section 333 that stores the block contrast value information about the reference image, the feature quantity calculation section 334 that calculates the feature quantity of each of the plurality of blocks, the effective block determination section 335 that determines whether or not each of the plurality of blocks is the effective block that is effective for the focus control process, based on the feature quantity, and acquires the determination results as the effective block information, the effective block information storage section 336 that stores the effective block information about the reference image, the AF area setting section 337 that sets the AF area from the effective block information about the reference image and the effective block information about the input image, and the contrast value determination section 338 that determines the image contrast value of the reference image from the AF area and the block contrast value information about the reference image, and determines the image contrast value of the input image from the AF area and the block contrast value information about the input image, the AF area setting section 337 setting a set of blocks that have been determined to be the effective block with respect to both the reference image and the input image, to be the AF area (see FIG. 2).

This makes it possible to implement an AF contrast value calculation process that utilizes the effective block information about the input image, and the effective block information about the reference image that is the target of comparison with the input image. Therefore, even when the position or the size of a bright spot within the image has changed during the AF process, identical contrast value calculation conditions can be employed for the comparison target images while appropriately excluding a bright spot area and the like, and it is possible to implement an appropriate AF control process.

The focus control section 330 may perform a reference image update process that sets the input image to be the reference image when the image contrast value of the input image is larger than the image contrast value of the reference image. The focus control section 330 may determine that the peak has been detected when the image contrast value of the input image is smaller than a threshold value that is calculated from the image contrast value of the reference image.

This makes it possible to detect the peak position of the contrast value (see FIG. 9). In the first embodiment, the reference image is an image among the processed images that is considered to be closest to the peak position. A case where the image contrast value of the input image is larger than the image contrast value of the reference image corresponds to the state 1 illustrated in FIG. 9. In this case, the reference image is updated since it is considered that the input image is closer to the peak position. A case where the image contrast value of the input image is smaller than the image contrast value of the reference image corresponds to the state 2 illustrated in FIG. 9. In this case, it is considered that the peak position is a position corresponding to the reference image. Note that a case where the image contrast value of the input image is smaller to only a small extent than the image contrast value of the reference image may have occurred due to the effects of noise or the like, and may not appropriately reflect the focus state. Therefore, it is desirable to determine that the peak has been detected on condition that the image contrast value of the input image is smaller to a certain extent than the image contrast value of the reference image. In the first embodiment, the determination process in the step S108 in FIG. 4 is performed using the coefficient K that satisfies "0<K<1".

The focus control section 330 may transmit an instruction to the lens position control section 340 when the focus control section 330 has determined that the peak has been detected, the instruction instructing to move the focus lens 240 to the focus lens position that corresponds to the reference image at a timing at which the focus control section 330 has determined that the peak has been detected.

This makes it possible to detect the position lensPosRef2 illustrated in FIG. 9 as the peak, and move the focus lens 240 to the position lensPosRef2. In this case, since the focus lens 240 is moved to the position at which the reference image was acquired, it is desirable to reduce the moving width (scan pitch) of the focus lens 240 to some extent (e.g., to such an extent that no problem occurs even if the peak is present between a given lens position and the subsequent lens position) in order to improve the AF accuracy.

The focus control section 330 may perform the focus control process that moves the focus lens position from the first position toward the second position by a given moving width, sets the image generated when the focus lens position is set to the first position to be the reference image, and sequentially sets the images generated while the focus lens position is moved from the first position to the second position by the given moving width to be the input image.

This makes it possible to implement the single AF process illustrated in FIG. 9. The first position may be the position of one end of the movable range of the focus lens 240, and the second position may be the position of the other end of the movable range of the focus lens 240. Note that the first position and the second position are not limited thereto.

The focus control section 330 may set a first reference image and a second reference image acquired at a timing that precedes the acquisition timing of the first reference image to be the reference image, and the AF area setting section 337 may set a set of blocks that have been determined to be the effective block with respect to all of the first reference image, the second reference image, and the input image, to be the AF area.

This makes it possible to appropriately compare the contrast values of three images (or four or more images). When a first comparison process on an image A and an image B and a second comparison process on the image B and an image C are performed separately, the image contrast value calculated by each comparison process may be effective for only comparison between two images, and it may be useless to compare the contrast value of the image A calculated by the first comparison process with the contrast value of the image C calculated by the second comparison process. It may be useful to compare the contrast value of the image A calculated by the first comparison process with the contrast value of the image C calculated by the second comparison process if the AF area is identical between the first comparison process and the second comparison process. However, such a case is limited. Therefore, it is desirable to use the effective block information about each image when performing the comparison process on three or more images.

The focus control section 330 may perform the reference image update process that sets the first reference image to be the second reference image, and sets the input image to be the first reference image when the image contrast value of the input image is larger than the image contrast value of the first reference image. The focus control section 330 may determine that the peak has been detected when the image contrast value of the input image is smaller than a threshold value that is calculated from the image contrast value of the first reference image.

The focus control section 330 may perform an interpolation process based on the image contrast values of the first reference image, the second reference image, and the input image at a timing at which the focus control section 330 has determined that the peak has been detected to calculate a maximum value when the focus control section 330 has determined that the peak has been detected, and transmit an instruction that instructs to move the focus lens 240 to the focus lens position that corresponds to the maximum value to the lens position control section 340.

This makes it possible to detect the peak position using the interpolation process (see FIG. 10). In thus case, even if an image has not been acquired (and the subsequent contrast value calculation process and the like have not been performed) at the peak position, the peak position can be detected as the peak. Therefore, since the moving width of the focus lens 240 can be increased as compared with the case illustrated in FIG. 9, it is possible to reduce the AF processing load, and implement a high-speed AF control process. Since the effective block information about three or more images is used, it is possible to appropriately calculate the relative relationship between the image contrast values, and appropriately implement the interpolation process.

The focus control section 330 may perform the focus control process that moves the focus lens position from the first position toward the second position by a given moving width, sets the image generated when the focus lens position is set to the first position to be the second reference image, sets the image generated when the focus lens position is set to the position that immediately follows the first position to be the first reference image, and sequentially sets the images generated while the focus lens position is moved from the position that immediately follows the position that immediately follows the first position to the second position by the given moving width to be the input image.

This makes it possible to implement the single AF process illustrated in FIG. 10.

The effective block determination section 335 may determine whether or not each block is the effective block by performing at least one of a first determination process that determines whether or not a bright spot is included in the block, a second determination process that determines whether or not the block is a dark area, and a third determination process that determines whether or not a treatment tool for tissue is included in the block.

This makes it possible to determine whether or not each block is the effective block based on a bright spot, a dark area, and a treatment tool (e.g., forceps). In this case, the maximum brightness value may be used as the feature quantity when implementing the first determination process, the average brightness value may be used as the feature quantity when implementing the second determination process, and the average Cr value and the average Cb value may be used as the feature quantity when implementing the third determination process. Note that another value may be used as the feature quantity when implementing each determination process. Whether or not each block is the effective block may be determined using a determination process other than the first determination process, the second determination process, and the third determination process.

The imaging device may include a distance measurement section (not illustrated in FIG. 1) that calculates distance information about the distance to the object based on the in-focus lens position detected by the focus control section 330.

This makes it possible to acquire the distance information about the distance to the object using the results of the autofocus control process performed by the focus control section 330. When the AF process has been completed by the focus control process, a system that includes the lens (e.g., focus lens 240), the image plane (e.g., the plane of the image sensor 260 in a narrow sense), and the object is in an in-focus state. The basic characteristics of the focus lens 240 and the image sensor 260 in an in-focus state can be acquired in advance as design items. Specifically, the position (in-focus object plane position) of the object point in an in-focus state can be calculated by referring to table data and the like provided that the position (in-focus lens position) of the focus lens 240 and the like in an in-focus state have been determined, and the in-focus object plane position is information that represents the distance to the captured object.

### 3. Second embodiment

An imaging device (endoscope system) according to the second embodiment of the invention is described below. The AF control section 339 according to the second embodiment performs a full-time AF process. Note that the configuration of the endoscope system according to the second embodiment is the same as described above in connection with the first embodiment except for the AF control section 339.

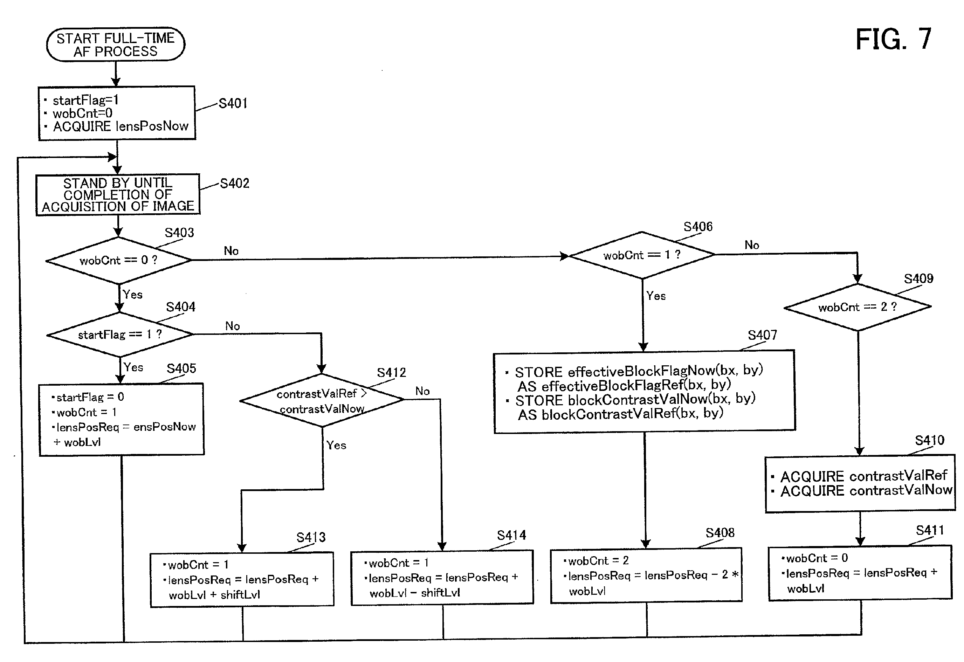

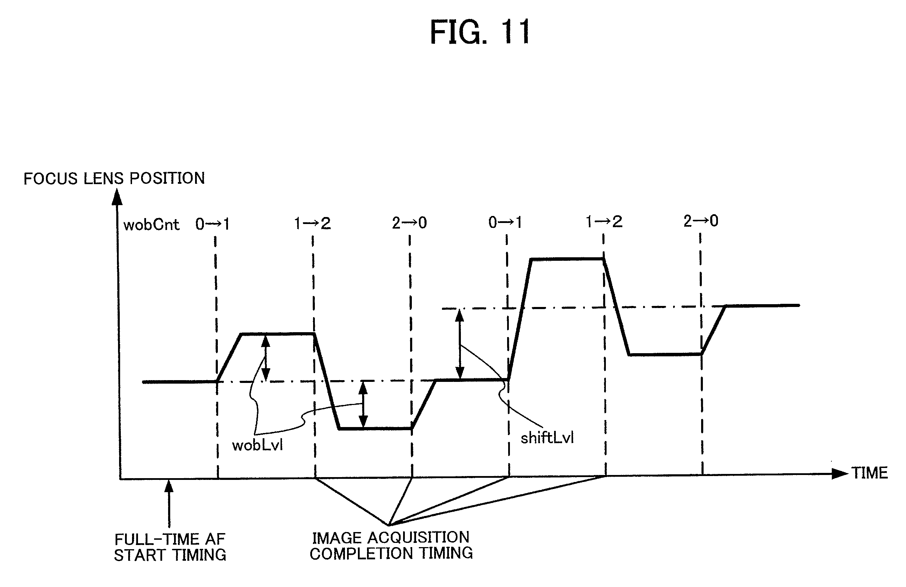

The operation of the AF control section 339 according to the second embodiment is described below with reference to FIG. 7 (flowchart). When the AF start signal has been output from the control section 360, the AF control section 339 sets a counter value wobCnt to "0", and sets a full-time AF start flag startFlag to "1" at a +timing at which the current image has been acquired. The AF control section 339 acquires the focus lens position lensPosNow from the lens position control section 340 (S401). Since the counter value wobCnt is set to "0", and the full-time AF start flag startFlag is set to "1" (Yes in S403 and S404), the AF control section 339 sets the full-time AF start flag startFlag to "0", and sets the counter value wobCnt to "1" (S405). The full-time AF start flag startFlag remains set to 0 thereafter. The AF control section 339 calculates the required lens position lensPosReq by calculating "lensPosReq=lensPosNow+wobLvl", and outputs the required lens position lensPosReq to the lens position control section 340. Note that wobLvl is the wobbling width of the focus lens 240 (see FIG. 11).

Since the counter value wobCnt is set to "1" (No in S403 and Yes in S406), the AF control section 339 outputs a control signal at a timing at which the current image has been acquired so that the effective block determination flag effectiveBlockFlagNow(bx, by) that is output from the effective block determination section 335 to the effective block information storage section 336 is stored as the effective determination flag effectiveBlockFlagRef(bx, by). The AF control section 339 outputs a control signal so that the contrast value blockContrastValNow(bx, by) that is output from the contrast value calculation section 332 to the contrast value storage section 333 is stored as the contrast value blockContrastValRef(bx,by) (S407). Note that the current image in this case is an image acquired when wobbling is performed in the direction in which the focus lens position increases (see FIG. 11). The AF control section 339 sets the counter value wobCnt to "2", calculates the required lens position lensPosReq by calculating "lensPosReq=lensPosNow-2*wobLvl", and outputs the required lens position lensPosReq to the lens position control section 340 (S408).

Since the counter value wobCnt is set to "2" (No in S403 and S406), the AF control section 339 acquires the contrast value contrastValNow and the contrast value contrastValRef output from the contrast value determination section 338 at a timing at which the current image has been acquired (S410). In this case, the AF control section 339 calculates the AF area from the effective block determination flag effectiveBlockFlagNow(bx,by) that has been acquired, and the effective determination flag effectiveBlockRef(bx,by) stored in the step S407, for example. Note that the current image in this case is an image acquired when wobbling is performed in the direction in which the focus lens position decreases (see FIG. 11). The AF control section 339 sets the counter value wobCnt to "0", calculates the required lens position lensPosReq by calculating "lensPosReq=lensPosNow+wobLvl", and outputs the required lens position lensPosReq to the lens position control section 340 (S411). Therefore, the focus lens position is returned to the wobbling center position.

Since the counter value wobCnt is set to "0", and the full-time AF start flag startFlag is set to "0" (Yes in S403 and No in S404), the AF control section 339 compares the contrast value contrastValNow with the contrast value contrastValRef at a timing at which the current image has been acquired (S412). When the contrast value contrastValRef is larger than the contrast value contrastValNow (Yes in S412), it is considered the in-focus lens position is situated in the direction in which the focus lens position increases. Therefore, the AF control section 339 sets the counter value wobCnt to "1", calculates the required lens position lensPosReq by calculating "lensPosReq=lensPosNow+wobLvl+shiftLvl", and outputs the required lens position lensPosReq to the lens position control section 340 (S413). Therefore, the wobbling center position is moved in the direction in which the focus lens position increases (see FIG. 11).

When the contrast value contrastValRef is smaller than the contrast value contrastValNow (No in S412), it is considered the in-focus lens position is situated in the direction in which the focus lens position decreases. Therefore, the AF control section 339 sets the counter value wobCnt to "1", calculates the required lens position lensPosReq by calculating "lensPosReq=lensPosNow+wobLvl-shiftLvl", and outputs the required lens position lensPosReq to the lens position control section 340 (S414). Therefore, the wobbling center position is moved in the direction in which the focus lens position decreases.

The AF control section 339 gradually brings the focus lens position closer to the in-focus lens position by continuously performing the above operation, and the focus lens position finally reaches the in-focus lens position. Even when the object has become out of focus due to the movement of the object or the like, the object can be brought into focus again by continuously performing the above operation.

The imaging device according to the second embodiment can thus correctly perform the full-time AF process even when the position of the exclusion target object within the image has changed during the AF process (e.g., when the position of a bright spot within the image has changed due to a small motion (movement) of tissue (object) or the like, or the user has moved forceps).

According to the second embodiment, the focus control section 330 included in the imaging device sets the image acquired at a timing at which the focus lens 240 has moved in a first direction with respect to a given center position to be the reference image, and sets the image acquired at a timing at which the focus lens 240 has moved in a second direction with respect to the center position to be the input image, the second direction differing from the first direction. The focus control section 330 performs a center position update process that updates the center position based on the comparison process that compares the image contrast value of the reference image with the image contrast value of the input image.

This makes it possible to implement the full-time AF process illustrated in FIG. 11 that continuously performs the AF control process. Note that the center position is the focus lens position when the counter value (wobCnt) is "0". The wobbling operation moves the focus lens position in the longitudinal direction by a given moving width (wobLvl) with respect to the center position. Specifically, the image acquired when the focus lens 240 is moved in one direction is compared with the image acquired when the focus lens 240 is moved in the other direction. When a series of operations has been completed (i.e., when the counter value wobCnt has changed in order of "0", "1", and "2", and returned to "0"), the reference image and the input image are newly acquired, and are not affected by the reference image and the input image acquired by the preceding series of operations. Note that the information about the preceding reference image and the like is not used for the process, but may be stored.

The focus control section 330 may perform the center position update process that moves the center position in the first direction by a given shift level when the image contrast value of the reference image is larger than the image contrast value of the input image, and may perform the center position update process that moves the center position in the second direction by the shift level when the image contrast value of the reference image is equal to or smaller than the image contrast value of the input image.

This makes it possible to update the wobbling center position based on the results of the image contrast value comparison process. Specifically, since it is estimated that the in-focus lens position corresponds to the reference image or the input image that has a larger contrast value, the focus lens 240 is moved in the corresponding direction by the shift level (shiftLvl).

### 4. Third embodiment

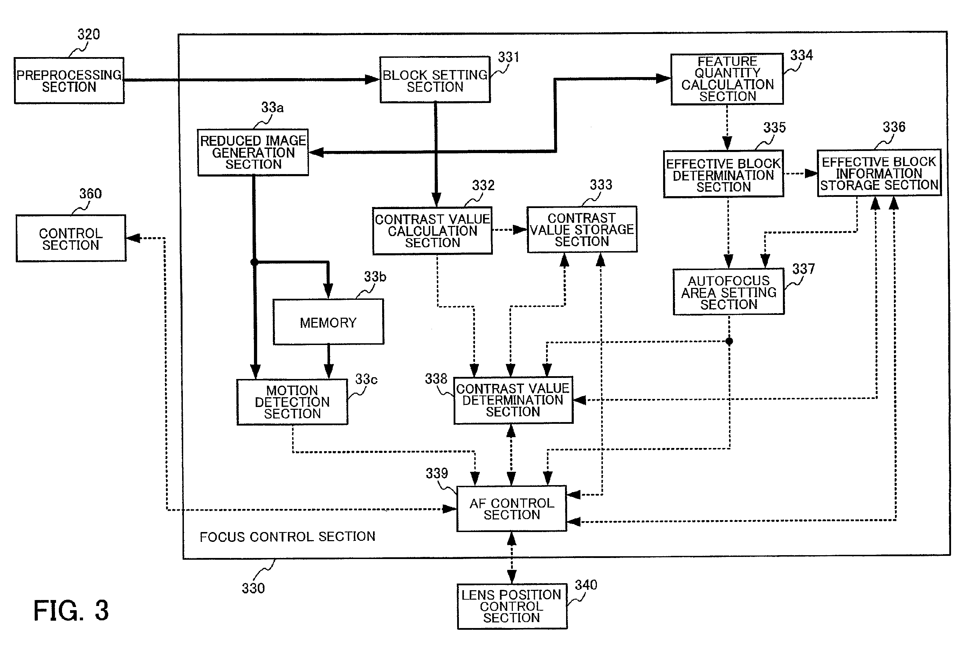

An imaging device (endoscope system) according to the third embodiment of the invention is described below with reference to FIG. 3. Note that the configuration of the endoscope system according to the third embodiment is the same as described above in connection with the first embodiment except for the focus control section 330.

The focus control section 330 according to the third embodiment includes a block setting section 331, a contrast value calculation section 332, a contrast value storage section 333, a feature quantity calculation section 334, an effective block determination section 335, an effective block information storage section 336, an AF area setting section 337, a contrast value determination section 338, an AF control section 339, a reduced image generation section 33a, a memory 33b, and a motion detection section 33c. Note that the configuration of the block setting section 331, the contrast value calculation section 332, the contrast value storage section 333, the feature quantity calculation section 334, the effective block determination section 335, the effective block information storage section 336, the AF area setting section 337, and the contrast value determination section 338 is the same as described above in connection with the first embodiment.

The reduced image generation section 33a generates a reduced image of each evaluation block that has been set by the block setting section 331, and outputs the reduced image to the memory 33b and the motion detection section 33c. For example, when the number of pixels included in the evaluation block b(bx, by) is N×N (see FIG. 12A), the reduced image generation section 33a averages the Y signal pixel values of M×M pixels (M is a divisor of N) to calculate the pixel value of a reduced image smallB(bx, by) of the evaluation block b(bx, by). The reduced image smallB(bx, by) that includes N/M×N/M pixels (see FIG. 12B) can be generated by performing the above process on all of the pixels included in the evaluation block b(bx, by). Note that FIG. 12A illustrates an example in which N=8 and M=4.

The memory 33b stores the reduced image output from the reduced image generation section 33a, and outputs the reduced image to the motion detection section 33c. The motion detection section 33c calculates the motion amount blockSAD(bx, by) of each evaluation block from the reduced image of the current image that is output from the reduced image generation section 33a, and the reduced image of the image that immediately precedes the current image that is output from the memory 33b, and outputs the motion amount blockSAD(bx, by) to the AF control section 339. Specifically, the motion detection section 33c calculates the difference between the corresponding pixels using the reduced image smallB1(bx, by) of each evaluation block of the current image and the reduced image smallB2(bx, by) of each evaluation block of the image that immediately precedes the current image, and calculates the motion amount blockSAD(bx, by) by calculating the sum of the differences corresponding to all of the pixels included in the reduced image of each evaluation block.

In the third embodiment, the motion amount blockSAD(bx, by) is calculated from the reduced image of each evaluation block in order to prevent a situation in which the motion amount blockSAD(bx, by) changes to a large extent depending on the degree of in-focus of the current image and the image that immediately precedes the current image. When the effect of the degree of in-focus of the current image and the image that immediately precedes the current image is small, the motion amount blockSAD(bx, by) may be calculated from the pixel values of each evaluation block in the same manner as described above without generating the reduced image.

The operation of the AF control section 339 when the AF control section 339 performs a single AF process is described below. When the AF start signal has been output from the control section 360, the AF control section 339 outputs the position (lens position A) of an arbitrary end of the movable range of the focus lens 240 to the lens position control section 340 as the required lens position, for example. When the focus lens 240 has reached the lens position A, the AF control section 339 changes the required lens position to the position (lens position B) of the other end of the movable range of the focus lens 240, and causes the focus lens 240 to start a scan motion to detect the in-focus lens position (peak detection process).

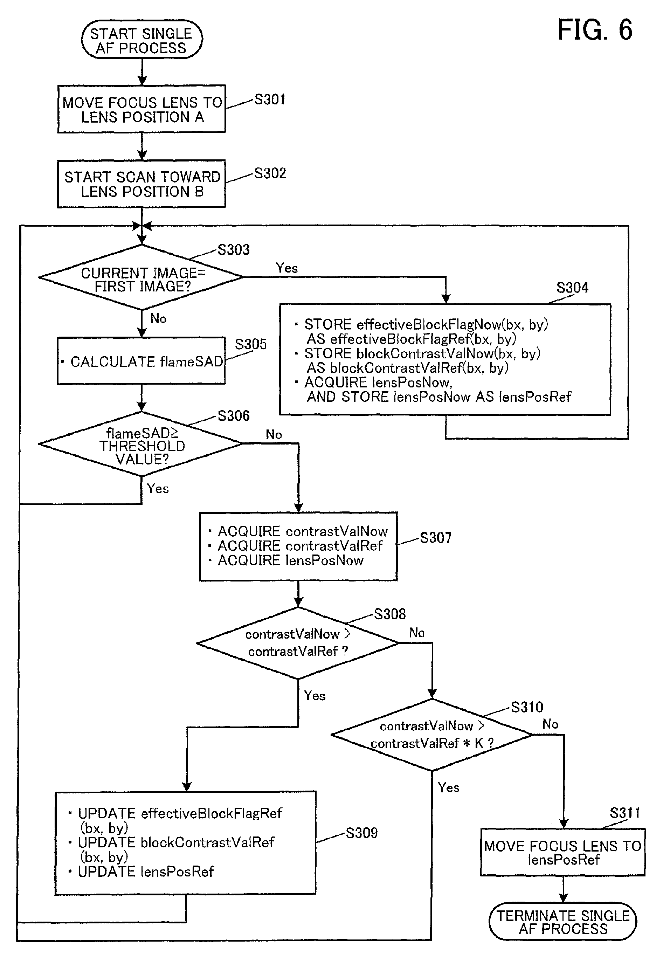

The operation of the AF control section 339 when the AF control section 339 performs the peak detection process is described below with reference to FIG. 6 (flowchart). When the current image is the first image after the scan motion has started (S301 to S304), the process is performed in the same as described above in connection with the first embodiment (S101 to S104 in FIG. 4). When the current image is the second or subsequent image after the scan motion has started (No in S303), the AF control section 339 calculates the motion amount flameSAD of the current image from the motion amount blockSAD(bx, by) output from the motion detection section 33c, and the AF area flag afAreaFlag(bx,by) output from the AF area setting section 337 (S305). Specifically, the AF control section 339 calculates the average value of the motion amounts blockSAD(bx, by) corresponding to the evaluation block for which the AF area flag afAreaFlag(bx, by) is set to "1", and sets the calculated value to be the motion amount flameSAD.

The AF control section 339 compares the calculated motion amount flameSAD with a given threshold value (motion amount threshold value) (S306). When the motion amount flameSAD is larger than the threshold value (Yes in S306), the AF control section 339 determines that the motion of the current image with respect to the image that immediately precedes the current image is large, and continues the peak detection process without performing the subsequent process (see FIG. 6). Specifically, when the motion of the current image is large, the contrast value contrastValNow may decrease due to a motion blur, and it may be determined that the in-focus lens position has been detected although the in-focus lens position has not been reached.

When the motion amount flameSAD is smaller than the threshold value (No in S306), the AF control section 339 continues the peak detection process in the same manner as described above in connection with the first embodiment. Note that the steps S307 to S311 respectively correspond to the steps S105 to S109 in FIG. 4.

After completion of the peak detection process, the AF control section 339 outputs the focus lens position lensPosRef to the lens position control section 340 as the required lens position, and terminates the single AF process when the focus lens 240 has reached the focus lens position lensPosRef (i.e., when the focus operation has been completed).

The endoscope system according to the third embodiment can thus perform a stable single AF process without detecting a wrong in-focus lens position, even when tissue (object) has moved to a large extent during the AF process.

When the motion amount flameSAD is larger than the threshold value, the AF control section 339 continues the peak detection process without performing the subsequent process. In this case, the focus lens position may be changed, or may not be changed. When the AF control section 339 continues the peak detection process after changing the focus lens position, it is possible to implement a high-speed focus control process since the subsequent process can be performed without performing the focus control process again corresponding to the focus lens position at which the image with a large motion was acquired. In this case, when the focus lens 240 is moved by a small moving amount, the accuracy of the in-focus lens position that is finally calculated is not affected to a large extent even if the process corresponding to one focus lens position is skipped. Specifically, it is desirable to update the focus lens position when the motion amount flameSAD is larger than the threshold value, and the process has been skipped, provided that the focus lens 240 is moved by a small moving amount in order to improve accuracy (see the basic method according to the first embodiment).

On the other hand, when the focus lens 240 is moved by a large moving amount, the accuracy of the in-focus lens position that is finally calculated is affected to a large extent since the blank width in which the contrast value is not acquired increases when the process corresponding to one focus lens position is skipped. Specifically, it is desirable to perform the process again corresponding to the same focus lens position without updating the focus lens position when the motion amount flameSAD is larger than the threshold value, and the process has been skipped, provided that the focus lens 240 is moved by a large moving amount (see the modification of the first embodiment).

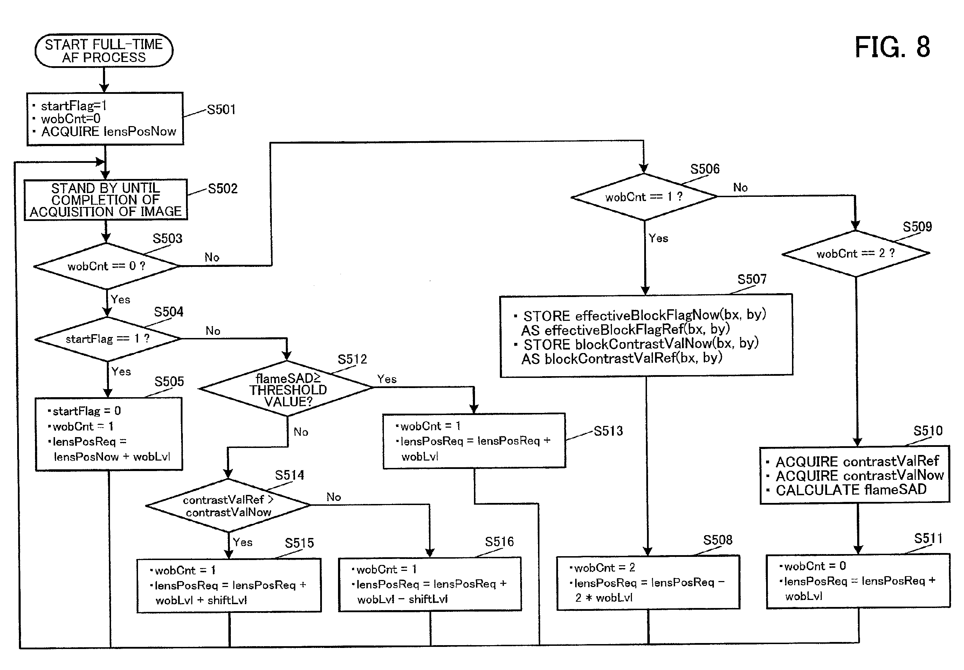

The process that utilizes the motion amount may also be combined with the full-time AF process described above in connection with the second embodiment instead of combining the process that utilizes the motion amount with the single AF process. The operation of the AF control section 339 when the AF control section 339 performs the full-time AF process is described below with reference to FIG. 8 (flowchart).

When the AF start signal has been output from the control section 360, the AF control section 339 sets the counter value wobCnt to "0", and sets the full-time AF start flag startFlag to "1" at a timing at which the current image has been acquired. The AF control section 339 performs the subsequent process in the same manner as described above in connection with the second embodiment. When the counter value wobCnt is set to "1", the AF control section 339 performs the subsequent process in the same manner as described above in connection with the second embodiment.

When the counter value wobCnt is set to "2", the AF control section 339 acquires the contrast value contrastValNow and the contrast value contrastValRef output from the contrast value determination section 338 at a timing at which the current image has been acquired. The AF control section 339 calculates (and stores) the motion amount flameSAD from the current image and the image that immediately precedes the current image in the same manner as described above. Note that the current image and the image that immediately precedes the current image refer to images acquired when the focus lens position has decreased or increased by the wobbling width wobLvl with respect to the wobbling center position. Specifically, the above process (S501 to S511) is performed in the same as described above in connection with the second embodiment (S401 to S411 in FIG. 7), except that the motion amount flameSAD is calculated in the step S510.

Since the counter value wobCnt is set to "0", and the full-time AF start flag startFlag is set to "0", the AF control section 339 compares the motion amount flameSAD with a given threshold value at a timing at which the current image has been acquired (S512). When the motion amount flameSAD is larger than the threshold value (Yes in S512), the AF control section 339 determines that the motion of the image during wobbling is large, sets the counter value wobCnt to "1", calculates the required lens position lensPosReq by calculating "lensPosReq=lensPosNow+wobLvl", and outputs the required lens position lensPosReq to the lens position control section 340 (S513) (see FIG. 8). Specifically, when the motion of the image during wobbling is large, the contrast value contrastValNow may decrease due to a motion blur, and the focus lens position may be moved in the direction opposite to the in-focus lens position. Therefore, the wobbling center position update process is skipped. When the motion amount flameSAD is smaller than the threshold value (No in S512), the AF control section 339 performs the process (S514 to S516) in the same manner as described above in connection with the second embodiment (S412 to S414 in FIG. 7).

The imaging device according to the third embodiment can thus perform a stable full-time AF process without moving the focus lens position in a wrong direction, even when tissue (object) has moved to a large extent during wobbling.

According to the third embodiment, the imaging device includes the motion detection section 33c that calculates the motion amount between the input image and the image acquired at a timing that immediately precedes the acquisition timing of the input image, and the focus control section 330 skips the process on the input image when the motion amount is larger than a given motion amount threshold value (see FIG. 3).

The motion amount may be calculated using various methods. For example, the motion amount may be calculated using the degree of difference (e.g., SAD or SSD) between the input image and the image acquired at a timing that immediately precedes the acquisition timing of the input image. Specifically, the motion amount blockSAD(bx, by) (degree of difference) of each block that is set within the input image may be calculated using the input image and the image that immediately precedes the input image, and the sum of the motion amounts blockSAD(bx, by) of blocks included in the AF area that has been set using the effective block information may be used as the motion amount of the input image, for example.

This makes it possible to skip the AF control process using the input image when the motion amount of the input image is large. It is likely that a motion blur has occurred when the motion amount of the input image is large, and the contrast value of the input image is small even if the focus lens position is appropriate. It is difficult to obtain appropriate results by performing the AF control process using such an input image. For example, when implementing the single AF process illustrated in FIG. 9, the state may be erroneously determined to the state 2 instead of the state 1, and the process may be terminated. Therefore, the AF control process is skipped when the motion amount of the input image is large.

The focus control section 330 may set an image that has been newly acquired at the focus lens position that corresponds to the input image to be the input image when the motion amount is larger than the motion amount threshold value. The focus control section 330 may instruct the lens position control section 340 to change the focus lens position when the motion amount is larger than the motion amount threshold value, and set an image that has been acquired after the focus lens position has been changed to be the input image.

This makes it possible to flexibly select the focus lens position when acquiring the subsequent input image after skipping the process on the input image having a large motion amount. Specifically, the focus lens position may be maintained, and the input image may be acquired at the same focus lens position, or the focus lens position may be updated, and the process may be performed on the input image that has been acquired at the updated focus lens position.

The imaging device may include the motion detection section 33c that calculates the motion amount between the input image and the image acquired at a timing that immediately precedes the acquisition timing of the input image, and the focus control section 330 may skip the process on the input image when the motion amount is larger than a given motion amount threshold value, and set an image generated after the focus lens position has been moved by the given moving width, to be the input image (see FIG. 3).

This makes it possible to skip the process on the input image when the motion amount of the input image is large, update the focus lens position, and set the image acquired at the updated focus lens position to be the input image when implementing the single AF process illustrated in FIG. 9. When implementing the single AF process illustrated in FIG. 9, it is considered that the focus lens 240 is moved by a small moving width in order to improve the accuracy of the AF control process. Therefore, the single AF process is not significantly affected even if the process is skipped at one focus lens position. Since the process is not performed again, it is possible to implement a high-speed AF process, for example.

The imaging device may include the motion detection section 33c that calculates the motion amount between the input image and the image acquired at a timing that immediately precedes the acquisition timing of the input image, and the focus control section 330 may skip the process on the input image without moving the focus lens position by a given moving width when the motion amount is larger than a given motion amount threshold value, and set an image generated at the focus lens position that corresponds to the input image to be the input image (see FIG. 3).

This makes it possible to skip the process on the input image and the focus lens position update process when the motion amount of the input image is large, and set the image acquired at the same focus lens position to be the input image when implementing the single AF process illustrated in FIG. 10. Since the peak position is calculated by the interpolation process when implementing the single AF process illustrated in FIG. 10, it is considered that the focus lens 240 is moved by a large moving width. Therefore, since the single AF process is affected to a large extent as compared with the single AF process illustrated in FIG. 9 when the process is skipped at one focus lens position, it is preferable to acquire the input image again at the same position.

The imaging device may include the motion detection section 33c that calculates the motion amount between the input image and the image acquired at a timing that immediately precedes the acquisition timing of the input image, and the focus control section 330 skips the center position update process when the motion amount is larger than a given motion amount threshold value (see FIG. 3).

This makes it possible to skip the center position update process when the motion amount of the input image is large when implementing the full-time AF process illustrated in FIG. 11. Since the full-time AF process compares two images, and moves the focus lens position in a better direction, it is preferable to acquire the image again at the current focus lens position without moving the focus lens position when it is difficult to perform a significant comparison since the contrast value is not appropriately calculated.

The first to third embodiments to which the invention is applied, and the modifications thereof, have been described above. Note that the invention is not limited to the first to third embodiments and the modifications thereof. Various modifications and variations may be made of the first to third embodiments and the modifications thereof without departing from the scope of the invention. A plurality of elements described above in connection with the first to third embodiments and the modifications thereof may be appropriately combined to implementing various configurations. For example, an arbitrary element may be omitted from the elements described above in connection with the first to third embodiments and the modifications thereof. Some of the elements described above in connection with the first to third embodiments and the modifications thereof may be appropriately combined. Any term cited with a different term having a broader meaning or the same meaning at least once in the specification and the drawings can be replaced by the different term in any place in the specification and the drawings. Specifically, various modifications and applications are possible without materially departing from the novel teachings and advantages of the invention.

### REFERENCE SIGNS LIST

100: light source section, 110: white light source, 120: condenser lens, 200: imaging section, 210: light guide fiber, 220: illumination lens, 230: objective lens system, 240: focus lens, 250: lens driver section, 260: image sensor, 300: processing section, 310: A/D conversion section, 320: preprocessing section, 330: focus control section, 331: block setting section, 332: contrast value calculation section, 333: contrast value storage section, 334: feature quantity calculation section, 335: effective block determination section, 336: effective block information storage section, 337: AF area setting section, 338: contrast value determination section, 339: AF control section, 33a: reduced image generation section, 33b: memory, 33c: motion detection section, 340: lens position control section, 350: image processing section, 360: control section, 400: display section, 500: external I/F section

## Claims

1. An imaging device comprising:
an optical system that includes a focus lens that adjusts an in-focus object plane position;
an image sensor that acquires an image of an object formed by the optical system;
a lens position control section that controls a focus lens position; and
a focus control section that performs an autofocus control process,
the focus control section including:
a block setting section that sets a plurality of blocks to an input image;
a contrast value calculation section that calculates block contrast value information that represents a contrast value of each of the plurality of blocks;
a contrast value storage section that stores the block contrast value information about a reference image;
a feature quantity calculation section that calculates a feature quantity of each of the plurality of blocks;
an effective block determination section that determines whether or not each of the plurality of blocks is an effective block based on the feature quantity, and acquires determination results as effective block information, the effective block being a block that is effective for the autofocus control process;
an effective block information storage section that stores the effective block information about the reference image;
an autofocus area setting section that sets an autofocus area from the effective block information about the reference image and the effective block information about the input image; and
a contrast value determination section that determines an image contrast value of the reference image from the autofocus area and the block contrast value information about the reference image, and determines the image contrast value of the input image from the autofocus area and the block contrast value information about the input image,
the autofocus area setting section setting a set of the blocks that have been determined to be the effective block with respect to both the reference image and the input image, to be the autofocus area.

2. The imaging device as defined in claim 1, further comprising:
a motion detection section that calculates a motion amount between the input image and the image acquired at a timing that immediately precedes an acquisition timing of the input image,
the focus control section skipping a process on the input image when the motion amount is larger than a given motion amount threshold value.

3. The imaging device as defined in claim 2,
the focus control section setting the image newly acquired at the focus lens position that corresponds to the input image, to be the input image when the motion amount is larger than the motion amount threshold value.

4. The imaging device as defined in claim 2,
the focus control section instructing the lens position control section to change the focus lens position when the motion amount is larger than the motion amount threshold value, and setting the image acquired after the focus lens position has been changed, to be the input image.

5. The imaging device as defined in claim 1,
the focus control section performing a reference image update process that sets the input image to be the reference image when the image contrast value of the input image is larger than the image contrast value of the reference image, and determining that a peak has been detected when the image contrast value of the input image is smaller than a threshold value that is calculated from the image contrast value of the reference image.

6. The imaging device as defined in claim 5,
the focus control section transmitting an instruction to the lens position control section when the focus control section has determined that the peak has been detected, the instruction instructing to move the focus lens to the focus lens position that corresponds to the reference image at a timing at which the focus control section has determined that the peak has been detected.

7. The imaging device as defined in claim 5,
the focus control section performing the autofocus control process that moves the focus lens position from a first position toward a second position by a given moving width, sets the image generated when the focus lens position is set to the first position to be the reference image, and sequentially sets the images generated while the focus lens position is moved from the first position to the second position by the given moving width to be the input image.

8. The imaging device as defined in claim 7, further comprising:
a motion detection section that calculates a motion amount between the input image and the image acquired at a timing that immediately precedes an acquisition timing of the input image,
the focus control section skipping a process on the input image when the motion amount is larger than a given motion amount threshold value, and setting the image generated after the focus lens position has been moved by the given moving width, to be the input image.

9. The imaging device as defined in claim 1,
the focus control section setting a first reference image and a second reference image acquired at a timing that precedes an acquisition timing of the first reference image, to be the reference image, and
the autofocus area setting section setting a set of the blocks that have been determined to be the effective block with respect to all of the first reference image, the second reference image, and the input image, to be the autofocus area.

10. The imaging device as defined in claim 9,
the focus control section performing a reference image update process that sets the first reference image to be the second reference image, and sets the input image to be the first reference image when the image contrast value of the input image is larger than the image contrast value of the first reference image, and determining that a peak has been detected when the image contrast value of the input image is smaller than a threshold value that is calculated from the image contrast value of the first reference image.

11. The imaging device as defined in claim 10,
the focus control section performing an interpolation process based on the image contrast values of the first reference image, the second reference image, and the input image at a timing at which the focus control section has determined that the peak has been detected, to calculate a maximum value when the focus control section has determined that the peak has been detected, and transmitting an instruction that instructs to move the focus lens to the focus lens position that corresponds to the maximum value, to the lens position control section.

12. The imaging device as defined in claim 9,
the focus control section performing the autofocus control process that moves the focus lens position from a first position toward a second position by a given moving width, sets the image generated when the focus lens position is set to the first position to be the second reference image, sets the image generated when the focus lens position is set to a position that immediately follows the first position to be the first reference image, and sequentially sets the images generated while the focus lens position is moved from a position that immediately follows the position that immediately follows the first position to the second position by the given moving width, to be the input image.

13. The imaging device as defined in claim 12, further comprising:
a motion detection section that calculates a motion amount between the input image and the image acquired at a timing that immediately precedes an acquisition timing of the input image,
the focus control section skipping a process on the input image without moving the focus lens position by the given moving width when the motion amount is larger than a given motion amount threshold value, and setting the image generated at the focus lens position that corresponds to the input image to be the input image.

14. The imaging device as defined in claim 1,
the focus control section setting the image acquired at a timing at which the focus lens has moved in a first direction with respect to a given center position, to be the reference image, and setting the image acquired at a timing at which the focus lens has moved in a second direction with respect to the center position, to be the input image, the second direction differing from the first direction, and
the focus control section performing a center position update process that updates the center position based on a comparison process that compares the image contrast value of the reference image with the image contrast value of the input image.

15. The imaging device as defined in claim 14,
the focus control section performing the center position update process that moves the center position in the first direction by a given shift level when the image contrast value of the reference image is larger than the image contrast value of the input image, and performing the center position update process that moves the center position in the second direction by the shift level when the image contrast value of the reference image is equal to or smaller than the image contrast value of the input image.

16. The imaging device as defined in claim 14, further comprising:
a motion detection section that calculates a motion amount between the input image and the image acquired at a timing that immediately precedes an acquisition timing of the input image,
the focus control section skipping the center position update process when the motion amount is larger than a given motion amount threshold value.

17. The imaging device as defined in claim 1,
the effective block determination section determining whether or not each of the plurality of blocks is the effective block by performing at least one of a first determination process that determines whether or not a bright spot is included in the block, a second determination process that determines whether or not the block is a dark area, and a third determination process that determines whether or not a treatment tool for tissue is included in the block.

18. The imaging device as defined in claim 1, further comprising:
a distance measurement section that calculates distance information about a distance to the object based on an in-focus lens position detected by the focus control section.

19. A method for controlling an imaging device comprising:
acquiring an input image;
setting a plurality of blocks to the input image;
calculating block contrast value information that represents a contrast value of each of the plurality of blocks;
calculating a feature quantity of each of the plurality of blocks, determining whether or not each of the plurality of blocks is an effective block based on the calculated feature quantity, and acquiring effective block information about the input image, the effective block being a block that is effective for a focus control process;
reading the block contrast value information about a reference image from a contrast value storage section, and reading the effective block information about the reference image from an effective block information storage section, the reference image being an image captured at a focus lens position that differs in in-focus object plane position with respect to the input image at a timing that precedes a capture timing of the input image;
setting a set of the blocks that have been determined to be the effective block with respect to both the reference image and the input image, to be an autofocus area;
calculating an image contrast value of the reference image from the autofocus area and the block contrast value information about the reference image, and calculating the image contrast value of the input image from the autofocus area and the block contrast value information about the input image; and
performing the focus control process based on a comparison process that compares the image contrast value of the reference image with the image contrast value of the input image.
